# EUROPÄISCHE PATENTSCHRIFT

(11) **EP 0 808 102 B1**
(45) Veröffentlichungstag und Bekanntmachung des Hinweises auf die Patenterteilung: **22.08.2001**
(21) Anmeldenummer: 96902938.8
(22) Anmeldetag: 30.01.1996
(51) Int. Cl.: A01N 37/26

(54) **FUNGIZIDES MITTEL**
FUNGICIDE
AGENT FONGICIDE

(30) Priorität: 11.02.1995 DE 19504599; 03.11.1995 DE 19540970
(43) Veröffentlichungstag der Anmeldung: 26.11.1997
(73) Patentinhaber: BASF AKTIENGESELLSCHAFT, 67056 Ludwigshafen (DE)
(72) Erfinder: WAGNER, Oliver, D-66450 Bexbach (DE); AMMERMANN, Eberhard, D-64646 Heppenheim (DE); LORENZ, Gisela, D-67434 Hambach (DE)
(86) Internationale Anmeldenummer: EP9600349
(87) Internationale Veröffentlichungsnummer: WO9624249

(56) Entgegenhaltungen:
- EP-A- 0 339 418
- EP-A- 0 608 739
- EP-A- 0 653 417
- EP-A- 0 720 980
- DATABASE WPI Section Ch, Week 9405 Derwent Publications Ltd., London, GB; Class C03, AN 94-040040 XP002004710 & JP,A,05 345 751 (NISSAN CHEM IND LTD) , 27.Dezember 1993 in der Anmeldung erwähnt

## Beschreibung

Die vorliegende Erfindung betrifft ein zur Bekämpfung von Schadpilzen geeignetes Mittel, enthaltend übliche Zusatzstoffe und eine wirksame Menge eines p-Hydroxyanilinderivates der allgemeinen Formel I 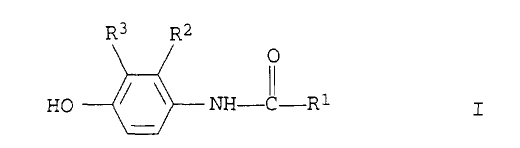 in der die Reste die folgende Bedeutung haben:
- R¹: C₆-C₁₅-Bicycloalkyl oder C₇-C₁₅-Bicycloalkenyl, wobei diese Reste partiell oder vollständig halogeniert sein und/oder eine bis fünf der folgenden Gruppen tragen können: C₁-C₄-Alkyl, C₁-C₄-Halogenalkyl, C₁-C₄-Alkoxy, C₁-C₄-Halogenalkoxy und Aryl, welches partiell oder vollständig halogeniert sein und/oder einen bis drei der folgenden Substituenten tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
- R²: und R³ unabhängig voneinander Halogen, C₁-C₄-Alkyl, C₁-C₄-Halogenalkyl, C₁-C₄-Alkoxy oder C₁-C₄-Halogenalkoxy.

Außerdem betrifft die Erfindung die Verwendung der Verbindungen I sowie der sie enthaltenden Mittel zur Bekämpfung von Schadpilzen sowie die Verwendung der Verbindungen I zur Herstellung der Mittel.

Aus der Literatur sind Alkylcarbonsäureanilide mit fungizider Wirkung bekannt (US-A 3 849 478, US-A 3 958 006, EP-A 293 718 und JP-A 345 751/93).

Aus der EP-A 339 418 sind weiterhin 4-Hydroxyanilide bekannt, welche jedoch hinsichtlich ihrer fungiziden Wirkung noch nicht befriedigen.

Der vorliegenden Erfindung lagen Mittel mit verbesserter Wirkung gegen ein breiteres Spektrum von Schadpilzen als Aufgabe zugrunde.

Demgemäß wurden die eingangs definierten Mittel gefunden.

Außerdem wurde die Verwendung dieser Mittel und der Verbindungen I zur Bekämpfung von Schadpilzen gefunden sowie die Verwendung der Verbindungen I zur Herstellung der Mittel.

Die Verbindungen der allgemeinen Formel I sowie deren Herstellung sind aus der EP-A 653 417 bzw. EP-A 653 418 bekannt. Die Verbindungen sind dort als Zwischenprodukte für fungizide O-acylierte p-Hydroxyanilinderivate beschrieben.

Man erhält die Hydroxyanilinderivate der allgemeinen Formel I, indem man ein p-Hydroxyanilin der Formel II in an sich bekannter Weise (vgl. DE-A 32 02 100; EP-A 339 418) in einem inerten organischen Lösungsmittel und gegebenenfalls in Gegenwart einer Base mit einem Carbonylderivat der Formel III umsetzt.

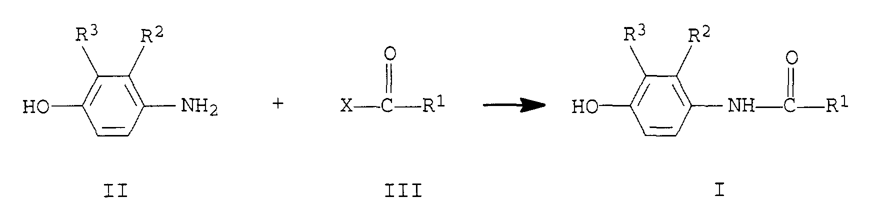

Die Variable X in der Formel III steht für Halogen, insbesondere Chlor, Brom und Jod oder eine andere bei Acylierungsreaktionen gebräuchliche Abgangsgruppe, z.B. R¹-CO-O.

Die Umsetzung erfolgt üblicherweise bei Temperaturen von -70 bis 140, vorzugsweise 0 bis 110°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid.

Es können auch Gemische der genannten Lösungsmittel verwendet werden.

Bevorzugte Lösungsmittel sind Dioxan, Tetrahydrofuran und Dimethylformamid, und zwar für sich allein oder im Gemisch.

Als Basen kommen in Betracht: Alkalimetall- und Erdalkalimetallhydroxide, z.B. Lithiumhydroxid, Natriumhydroxid, Kaliumhydroxid, Calciumhydroxid; Alkalimetall- und Erdalkalimetalloxide, z.B. Lithiumoxid, Natriumoxid, Calciumoxid, Magnesiumoxid; Alkalimetallamide, z.B. Lithiumamid, Natriumamid, Kaliumamid; Alkalimetall- und Erdalkalimetallcarbonate, z.B. Lithiumcarbonat, Calciumcarbonat; Alkalimetallhydrogencarbonate, z.B. Natriumhydrogencarbonat; Alkalimetall- und Erdalkalimetallalkoholate, z.B. Natriummethanolat, Natriumethanolat, Kaliumethanolat, Kalium- tert.-Butanolat, Dimethoxymagnesium; organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine.

Besonders bevorzugt werden Alkalimetallcarbonate, -alkoholate oder tertiäre Amine verwendet.

Die Basen werden im allgemeinen in äquimolaren Mengen eingesetzt, sie können aber auch im Überschuß oder als Lösungsmittel verwendet werden.

Die Edukte werden im allgemeinen in äquimolaren Mengen miteinander umgesetzt. Es kann für die Ausbeute vorteilhaft sein, das Carbonylderivat III in einem Über- oder Unterschuß bezogen auf das p-Hydroxyanilin II einzusetzen.

Die für die Umsetzung benötigten Ausgangsstoffe II und III sind aus der Literatur bekannt (II: J. Chem. Soc. PT I, 1, 1 (1973); Houben-Weyl, Vol. 10/1, s. 1140 f.; ibid. Vol. 6/1c, S. 85-101; III: Houben-Weyl, E5, Teil 1, S. 587; Can. J. Chem. 71, S. 1099-1105 (1993)) oder können gemäß der zitierten Literatur hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Produkte fallen z.T. in Form farbloser oder schwach bräunlicher, zäher Öle an, die unter vermindertem Druck und bei mäßig erhöhter Temperatur von flüchtigen Anteilen befreit oder gereinigt werden können. Sofern die Zwischen- und Endprodukte als Feststoffe erhalten werden, kann die Reinigung auch durch Umkristallisieren oder Digerieren erfolgen.

Die Verbindungen I können ein oder mehrere Asymmetriezentren enthalten und werden nach den beschriebenen Verfahren in Form von Enantiomeren- oder Diastereomerengemischen erhalten. Die Mengenverhältnisse können dabei in Abhängigkeit von den Gruppen unterschiedlich sein. Diese Gemische können ggf. nach üblichen Methoden getrennt werden. Die Verbindungen I können sowohl als reine Isomere oder auch als Isomerengemische zur Anwendung kommen.

Bei den eingangs angegebenen Definitionen der Verbindungen I wurden Sammelbegriffe verwendet, die allgemein repräsentativ für die folgenden Substituenten stehen:
Halogen: Fluor, Chlor, Brom und Jod;
Alkyl: geradkettige oder verzweigte Alkylgruppen mit 1 bis 3, 4 Kohlenstoffatomen, z.B. C₁-C₄-Alkyl wie Methyl, Ethyl, Propyl, 1-Methylethyl, Butyl, 1-Methyl-propyl, 2-Methylpropyl, 1,1-Dimethylethyl;
Halogenalkyl bzw. partiell oder vollständig halogeniertes Alkyl: geradkettige oder verzweigte Alkylgruppen mit 1 bis 4 Kohlenstoffatomen wie vorstehend genannt, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 3 oder 4 Kohlenstoffatomen, wie Methyloxy, Ethyloxy, Propyloxy, 1-Methylethyloxy, Butyloxy, 1-Methyl-propyloxy, 2-Methylpropyloxy und 1,1-Dimethylethyloxy;
Halogenalkoxy: geradkettige oder verzweigte partiell oder vollständig halogenierte Alkylgruppen mit 1 bis 4 Kohlenstoffatomen wie vorstehend genannt, wobei diese Gruppen über ein Sauerstoffatom (-O-) an das Gerüst gebunden sind;
Alkylthio: geradkettige oder verzweigte Alkylgruppen mit 1 bis 4 Kohlenstoffatomen wie vorstehend genannt, welche über ein Schwefelatom (-S-) an das Gerüst gebunden sind;
Bicycloalkyl: bicyclische Alkylgruppen mit 6 bis 15 Kohlenstoffringgliedern, z.B. Bicyclo-[2.1.1]-hex-5-yl, Bicyclo- [2.2.1] -hept-2-yl, Bicyclo- [2.2.2] oct-2-yl, Bicyclo- [3.2,1] -oct-6-yl, Bicyclo- [3.2.2] -non-6-yl, Bicyclo- [4.2.2] -dec-7-yl, Bicyclo-[3.1.0]-hex-1-yl, Bicyclo-[4.1.0]-hept-1-yl, Bicyclo-[4.3.0]-non-1-yl, Bicyclo- [4.4.0] -dec-1-yl, besonders bevorzugt 5-Methyl-bicyclo- [2,1,1] -hex-5-yl, 2-Methyl-bicyclo- [2.2,1] hept-2-yl, 2-Methyl-bicyclo- [2.2.2] oct-2-yl, 6-Methyl-bicyclo- [3.2.1] -oct-6-yl, 6-Methyl-bicyclo-[3.2.2]-non-6-yl, 7-Methyl-bicyclo-[4.2.2]-dec-7-yl, 1-Methyl-bicyclo- [3.1.0] -hex-1-yl, 1-Methyl-bicyclo-[4.1.0]-hept-1-yl, 1-Methyl-bicyclo-[4.3.0]-non-1-yl, 1-Methyl-bicyclo- [4.4.0] -dec-1-yl, 2-Methyl-bicyclo- [3,1.0] -hex-1-yl, 2-Methyl-bicyclo-[4.1.0]-hept-1-yl, 2-Methyl-bicyclo- [4.3.0] -non-1-yl, 2-Methyl-bicyclo-[4.4.0]-dec-1-yl;
Bicycloalkenyl: bicyclische Alkenylgruppen mit 7 bis 15 Kohlenstoffringgliedern, z.B. Bicyclo- [2.2,1] -hept-2-en-5-yl, Bicyclo-[2.2.2]-oct-2-en-5-yl, Bicyclo-[4.2.2] -dec-7-en-2-yl, Bicyclo-[4.3.0]-non-7-en-1-yl, Bicyclo-[4.4.0]-dec-3-en-1-yl, Bicyclo-[4.1.0] -hept-3-en-1-yl, 5-Methyl-bicyclo-[2.2.1]-hept-2-en-5-yl, 5-Methyl-bicyclo-[2.2.2]-oct-2-en-5-yl, 2-Methylbicyclo-[4.2.2]-dec-7-en-2-yl, 2-Methyl-bicyclo - [4.3.0]-non-7-en-1-yl, 2-Methyl-bicyclo-[4.4.0]-dec-3-en-1-yl, 2-Methyl-bicyclo- [4.1.0] -hept-3-en-1-yl;
Aryl: Phenyl oder Naphthyl.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Im Hinblick auf ihre biologische Wirkung gegen Schadpilze sind Verbindungen der Formel I bevorzugt,
- in denen R¹ für 2-Methyl-bicyclo- [2.2,1] -hept-2-yl, 2-Methylbicyclo-[2.2.2]-oct-2-yl, Bicyclo-[4.1.0]-hept-1-yl, 1-Methyl-bicyclo-[4.3.0]-non-1-yl, 1-Methyl-bicyclo-[4.4.0]-dec-1-yl, 1-Methyl-bicyclo-[4.1.0]-hept-1-yl, 2-Methyl-bicyclo- [4.3.0]-non-1-yl, 2-Methyl-bicyclo-[4.4.0]-dec-1-yl, 5-Methyl-bicyclo- [2.2.1] -hept-2-yl, 5-Methyl-bicyclo-[2.2.2]-oct-2-en-5-yl, 2-Methyl-bicyclo-[4.4.0]-dec-3-en-1-yl, oder 2-Methyl-bicyclo- [4.1.0] -hept-3-en-1-yl steht,
- in denen R² für Halogen, Alkyl oder Alkoxy steht,
- in denen R² für Alkyl, insbesondere Methyl, steht,
- in denen R² für Fluor oder Chlor steht,
- in denen R³ für Alkyl, Halogenalkyl, Alkoxy oder Halogenalkoxy steht,
- in denen R³ für Halogen, Alkyl, Halogenalkyl oder Halogenalkoxy steht und/oder
- in denen R³ für Fluor, Chlor, Methyl oder Trifluormethyl steht.

Ganz besonders bevorzugt sind im Hinblick auf ihre Verwendung zur Bekämpfung von Schadpilzen die in den anschließenden Tabellen 1 bis 8 zusammengestellten Verbindungen.

### Tabelle 1:

Verbindungen der allgemeinen Formel I.1, in denen die Kombination der Substituenten R² und R³ für eine Verbindung jeweils einer Zeile der Tabelle A steht

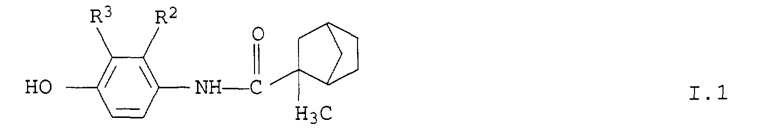

### Tabelle 2:

Verbindungen der allgemeinen Formel I.2, in denen die Kombination der Substituenten R² und R³ für eine Verbindung jeweils einer Zeile der Tabelle A steht

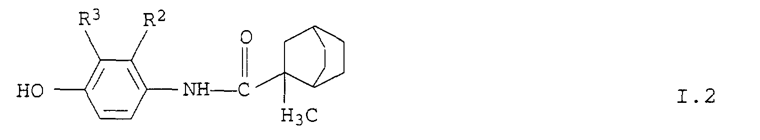

### Tabelle 3:

Verbindungen der allgemeinen Formel I.3, in denen die Kombination der Substituenten R² und R³ für eine Verbindung jeweils einer Zeile der Tabelle A steht

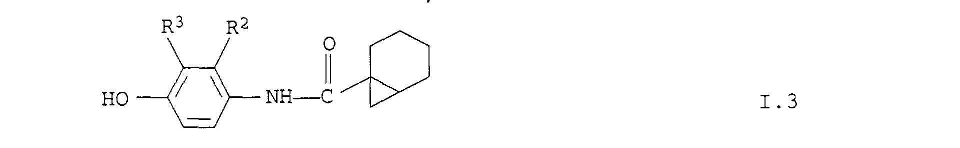

### Tabelle 4:

Verbindungen der allgemeinen Formel I.4, in denen die Kombination der Substituenten R² und R³ für eine Verbindung jeweils einer Zeile der Tabelle A steht

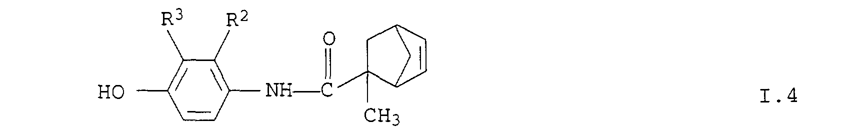

### Tabelle 5:

Verbindungen der allgemeinen Formel I.5, in denen die Kombination der Substituenten R² und R³ für eine Verbindung jeweils einer Zeile der Tabelle A steht

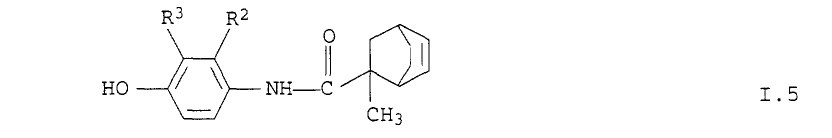

### Tabelle 6:

Verbindungen der allgemeinen Formel I.6, in denen die Kombination der Substituenten R² und R³ für eine Verbindung jeweils einer Zeile der Tabelle A steht

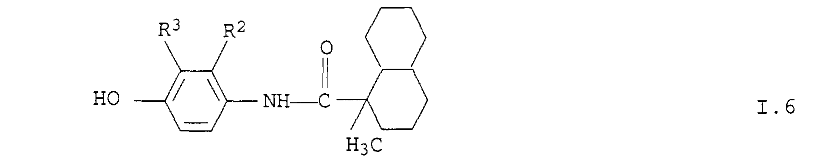

### Tabelle 7:

Verbindungen der allgemeinen Formel I.7, in denen die Kombination der Substituenten R² und R³ für eine Verbindung jeweils einer Zeile der Tabelle A steht

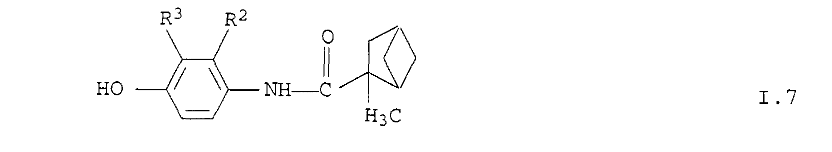

### Tabelle 8:

Verbindungen der allgemeinen Formel I.8, in denen die Kombination der Substituenten R² und R³ für eine Verbindung jeweils einer Zeile der Tabelle A steht

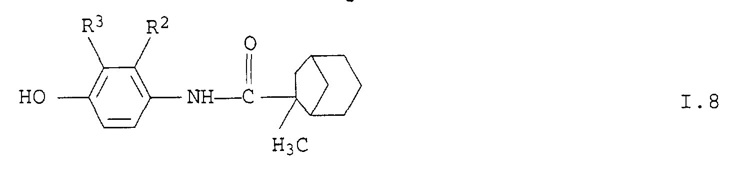

### Tabelle A

| Nr. | R² | R³ |
|---|---|---|
| 1 | Cl | Cl |
| 2 | Cl | CH₃ |
| 3 | CH₃ | Cl |
| 4 | F | F |
| 5 | F | CH₃ |
| 6 | CH₃ | F |
| 7 | Cl | F |
| 8 | F | Cl |
| 9 | CH₃ | CH₃ |

Die neuen Verbindungen der Formel I eignen sich als Fungizide.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B.

Cyclohexanon), Amine (z.B. Ethanolamin, Dimethylformamid) und Wasser; Trägerstoffe wie natürliche Gesteinsmehle (z.B. Kaoline, Tonerden, Talkum, kreide) und synthetische Gesteinsmehle (z.B. hochdisperse Kieselsäure, Silikate), Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, hepta- und octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, octyl- oder Nonylphenol, Alkylphenol-, Tributylphenylpolyglykolether, Alkylarylpolyetheralkohole, isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind (die verwendeten Wirkstoffe tragen die Bezeichnung gemäß Tabelle B):
I. eine Lösung aus 90 Gew.-Teilen der Verbindung Nr. 1 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 1, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 2, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 2, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 2 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 2, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 2, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 2, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-Harnstoff-Formaldehyd-Kondensates und 68 Gew.-Teilen eines paraffinischen Mineralöls.

Die Verbindungen I und die erfindungsgemäßen Mittel zeichnen sich durch eine hervorragende Wirksamkeit gegen ein breites Spektrum von pflanzenpathogenen Pilzen, insbesondere aus der Klasse der Ascomyceten, Basidiomyceten, Deuteromyceten und Phycomyceten aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen sowie an den Samen dieser Pflanzen.

Die Verbindungen I und die erfindungsgemäßen Mittel werden angewendet, indem man die Schadpilze, deren Lebensraum oder die vor Pilzbefall zu schützenden Saatgüter, Pflanzen, Flächen, Materialien oder Räume mit einer fungizid wirksamen Menge der Wirkstoffe I oder der Mittel behandelt.

Die Anwendung erfolgt vor oder nach der Infektion der Saatgüter, Materialien oder Pflanzen durch die Pilze.

Speziell eignen sich die Verbindungen I und die erfindungsgemäßen Mittel zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Besonders bevorzugt ist die Bekämpfung von Botrytis mittels der Verbindungen I oder der erfindungsgemäßen Mittel.

Die Verbindungen I und die erfindungsgemäßen Mittel können auch im Materialschutz (Holzschutz) eingesetzt werden, z.B. gegen Paecilomyces variotii.

Die erfindungsgemäßen Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können auch zusammen mit anderen Wirkstoffen vorliegen, z.B. mit Herbiziden, Insektiziden, Wachstumsregulatoren, Fungiziden oder auch mit Düngemitteln.

Beim Vermischen mit weiteren Fungiziden erhält man dabei in vielen Fällen eine Vergrößerung des fungiziden Wirkungsspektrums.

Die folgende Liste von fungiziden Wirkstoffen, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1- [bis- (dimethylamino)-phosphinyl]-3-phenyl-1,2,4-triazol, 2,3-Dicyano-1,4-dithioanthrachinon, 2-Thio-1,3-dithiolo- [4,5-b]-chinoxalin, 1- (Butylcarbamoyl) -2-benzimidazolcarbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbon säurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlorethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N- [3- (p-tert.- Butylphenyl) -2-methylpropyl] -cis-2,6-dimethylmorpholin, N- [3-(p-tert.-Butylphenyl) -2-methylpropyl] -piperidin, 1- [2- (2,4-Dichlorphenyl) -4-ethyl-1,3-dioxolan-2-yl-ethyl]-1H-1,2,4-triazol, 1- [2- (2,4-Dichlorphenyl) -4-n-propyl-1,3-dioxolan-2-yl-ethyl]-1H-1,2,4-triazol, N-(n-Propyl)-N-(2,4,6-trichlorphenoxyethyl) -N'-imidazol-yl-harnstoff, 1-(4-Chlorphenoxy)-3,3-dimethyl-1-(1H-1,2,4-triazol-1-yl) -2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-3-methoxycarbonyl-2-thioureido)-benzol, [2-(4-Chlorphenyl)ethyl]-(1,1-dimethylethyl)-1H-1,2,4-triazol-1-ethanol sowie
verschiedene Fungizide, wie Dodecylguanidinacetat, 3- [3- (3, 5-Dimethyl-2-oxycyclohexyl) -2-hydroxyethyl)] glutarimid, Hexachlorbenzol, DL-Methyl-N-(2,6-dimethyl-phenyl) -N-furoyl(2)-alaninat, DL-N-(2,6-Dimethyl-phenyl) -N-(2'-methoxyacetyl)-alanin-methylester, N-(2,6-Dimethylphenyl)-N-chloracetyl-D,L-2-aminobutyrolacton, DL-N-(2,6-Dimethylphenyl) -N- (phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl] -1,3-oxazolidin-2,4-dion, 3-(3,5-Dichlorhenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.
Strobilurine wie Methyl-E-methoximino- [α-(o-tolyloxy)-otolyl] -acetat, Methyl-E-2-{2-[6-(2-cyanophenoxy)pyrimidin-4-yloxy]-phenyl}-3-methoxyacrylat, Methyl-E-methoximino-[α-(2-phenoxyphenyl)]-acetamid, Methyl-E-methoximino-[α-(2,5-dimethylphenoxy)-o-tolyl]-acetamid.
Anilinopyrimidine wie N-(4,6-Dimethylpyrimidin-2-yl)-anilin, N-[4-Methyl-6-(1-propinyl)-pyrimidin-2-yl]-anilin, N-(4-Methyl-6-cyclopropyl-pyrimidin-2-yl)-anilin.

Phenylpyrrole wie 4-(2,2-difluor-1,3-benzodioxol-4-yl)-pyrrol-3-carbonitril.

Zimtsäureamide wie 3-(4-Chlorphenyl)-3-(3,4-dimethoxyphenyl)-acrylsäuremorpholid.

(2RS,3SR)-1[3-(2-Chlorphenyl)-2,3-epoxy-2-(4-fluorphenyl)propyl]-1H-1,2,4-triazol.

### Synthesebeispiele

Die im nachstehenden Synthesebeispiel wiedergegebene Vorschrift zur Herstellung der Verbindungen Ia kann unter Abwandlung der Ausgangsverbindungen zur Gewinnung weiterer Vertreter der Verbindungen I benutzt werden. Demgemäß hergestellte Beispiele sind in der anschließenden Tabelle B mit physikalischen Daten angegeben.

### 3-Chlor-2,2-dimethylpropancarbonsäure-N- (2,3-dichlor-4-hydroxyphenyl) amid

Zu einer Lösung von 1,93 g (0,011 mol) 4-Amino-2,3-dichlorphenol in 30 ml Tetrahydrofuran wurden bei 0°C 1,55 g (0,01 mol) Chlorpivalinsäurechlorid getropft und bei 25°C solange nachgerührt, bis dünnschichtchromatographisch keine Ausgangsverbindung mehr nachgewiesen werden konnte. Anschließend wurde das Reaktionsgemisch auf Wasser gegeben und dreimal mit 20 ml Methylenchlorid extrahiert. Die vereinigten organischen Phasen wurden zweimal mit Wasser gewaschen, getrocknet und eingeengt. Der Rückstand wurde an Kieselgel chromatographiert (Laufmittel: Cyclohexan: Essigester=2:1; Ausbeute: 0,75 g, Fp. 115°C).

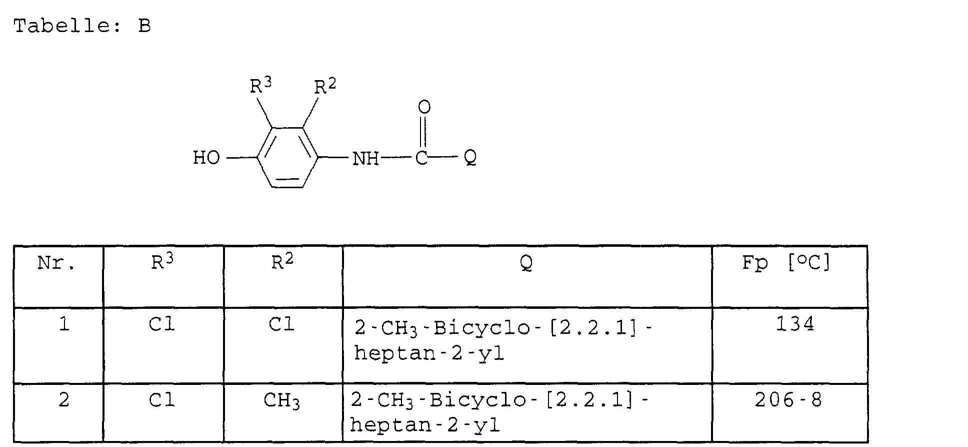

### Anwendungsbeispiele

Für die folgenden Versuche zur fungiziden Wirkung der Verbindungen I wurde eine Emulsion verwendet, welche zu 10 Gew.-% aus dem Wirkstoff und zu 90 Gew.-% aus einem Gemisch aus

| | |
|---|---|
| 70 Gew.-% | Cyclohexanol, |
| 20 Gew.-% | Nekanil® LN (Lutensol® AP6, Netzmittel mit Emulgierz-und Dispergierwirkung auf der Basis ethoxylierter Alkylphenole) und |
| 10 Gew.-% | Emulphor® EL (Emulan® EL, Emulgator auf der Basis ethoxylierter Fettalkohole) |

bestand. Die gewünschten Wirkstoff-Konzentrationen wurden durch Verdünnen dieser Emulsion mit Wasser eingestellt.

### Anwendungsbeispiel 1

### Wirksamkeit gegen Botrytis cinerea

Paprikasämlinge der Sorte "Neusiedler Ideal Elite" wurden, nachdem sich 4 bis 5 Blätter gut entwickelt hatten, mit wäßrigen Suspensionen, die 80 Gew.-% Wirkstoff und 20 Gew.-% Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten. Die Auswertung erfolgte visuell.

Der Versuch wurde mit jeweils einer der Verbindungen aus Tabelle B durchgeführt.

Der Pilzbefall, ausgedrückt in Prozent der Blattfläche, lag bei Applikation einer 250 ppm Wirkstoff enthaltenden Aufbereitung zwischen 0 und 5 %.

Unbehandelte Pflanzen zeigten einen Befall von 80 %.

### Anwendungsbeispiel 2

### Wirksamkeit gegen Weizenmehltau

Blätter von in Töpfen gewachsenen Weizenkeimlingen der Sorte "Frühgold" wurden mit wäßriger Spritzbrühe, die 80 Gew.-% Wirkstoff und 20 Gew.-% Emulgiermittel in der Trockensubstanz enthielten, besprüht und 24 Stunden nach dem Antrocken des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung visuell ermittelt.

Der Versuch wurde mit jeweils einer der folgenden Verbindungen aus Tabelle B durchgeführt.

Der Pilzbefall, ausgedrückt in Prozent der Blattfläche, lag bei Applikation einer 250 ppm Wirkstoff enthaltenden Aufbereitung zwischen 5 und 15 %.

Unbehandelte Pflanzen zeigten einen Befall von 70 %.

## Patentansprüche

1. Zur Bekämpfung von Schadpilzen geeignetes Mittel, enthaltend eine wirksame Menge eines p-Hydroxyanilinderivates der allgemeinen Formel I 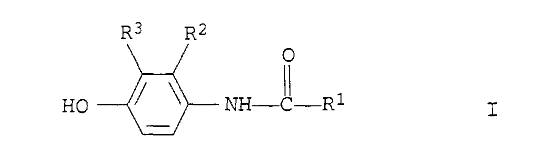 in der die Reste die folgende Bedeutung haben:
R¹ C₆-C₁₅-Bicycloalkyl oder C₇-C₁₅-Bicycloalkenyl, wobei diese Reste partiell oder vollständig halogeniert sein und/oder eine bis fünf der folgenden Gruppen tragen können: C₁-C₄-Alkyl, C₁-C₄-Halogenalkyl, C₁-C₄-Alkoxy, C₁-C₄-Halogenalkoxy und Aryl, welches partiell oder vollständig halogeniert sein und/oder einen bis drei der folgenden Substituenten tragen kann: Nitro, Cyano, C₁-C₄-Alkyl, C₁-C₄-Halogenalkyl, C₁-C₄-Alkoxy, C₁-C₄-Halogenalkoxy und C₁-C₄-Alkylthio;
R² und R³ unabhängig voneinander Halogen, C₁-C₄-Alkyl, C₁-C₄-Halogenalkyl, C₁-C₄-Alkoxy oder C₁-C₄-Halogenalkoxy.

2. Verwendung der Verbindungen der allgemeinen Formel I gemäß Anspruch 1 zur Bekämpfung von Schadpilzen.

3. Verwendung der Mittel gemäß Anspruch 1 zur Bekämpfung von Schadpilzen.

4. Verwendung der Verbindungen der allgemeinen Formel I gemäß Anspruch 1 zur Herstellung von zur Bekämpfung von Schadpilzen geeigneten Mitteln.

5. Verfahren zur Bekämpfung von Schadpilzen, dadurch gekennzeichnet, daß man die Schadpilze, deren Lebensraum oder die von ihnen freizuhaltenden Saatgüter, Pflanzen, Flächen, Mate. rialien oder Räume mit einer wirksamen Menge einer Verbindung der allgemeinen Formel I gemäß Anspruch 1 oder eines Mittels gemäß Anspruch 1 behandelt.

6. Verfahren nach Anspruch 5, dadurch gekennzeichnet, daß man die Behandlung mit einer Verbindung I aus der folgenden Tabelle oder einem diese Verbindung I enthaltenden Mittel gemäß Anspruch 1 vornimmt: 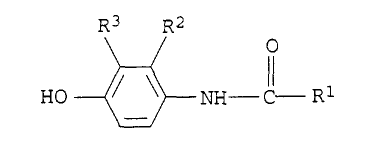
| Nr. | R³ | R² | R¹ |
|---|---|---|---|
| 1 | Cl | Cl | 2-CH₃-Bicyclo-[2.2.1]-heptan - 2 - yl |
| 2 | Cl | CH₃ | 2-CH₃-Bicyclo- [2.2.1] -heptan - 2 - yl |
| 3 | Cl | Cl | 2-CH₃-Bicyclo- [2.2.1]-hepten - 2 - yl |

## Claims

1. A composition which is suitable for controlling fungal pests, comprising an effective amount of a p-hydroxyaniline derivative of the general formula I 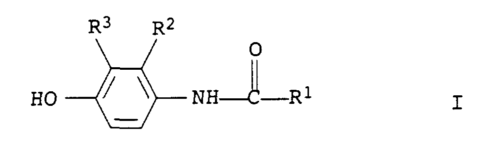 where the radicals have the following meanings:
R¹ C₆-C₁₅-bicycloalkyl or C₇-C₁₅-bicycloalkenyl, it being possible for these radicals to be partially or fully halogenated and/or to have attached to them one to five of the following groups: C₁-C₄-alkyl, C₁-C₄-haloalkyl, C₁-C₄-alkoxy, C₁-C₄-haloalkoxy and aryl which can be partially or fully halogenated and/or can have attached to it one to three of the following substituents: nitro, cyano, C₁-C₄-alkyl, C₁-C₄-haloalkyl, C₁-C₄-alkoxy, C₁-C₄-haloalkoxy and C₁-C₄-alkylthio;
R² and R³ independently of one another are halogen, C₁-C₄-alkyl, C₁-C₄-haloalkyl, C₁-C₄-alkoxy or C₁-C₄-haloalkoxy.

2. The use of the compounds of the general formula I as claimed in claim 1 for controlling fungal pests.

3. The use of the compositions as claimed in claim 1 for controlling fungal pests.

4. The use of the compounds of the general formula I as claimed in claim 1 for the preparation of compositions which are suitable for controlling fungal pests.

5. A method of controlling fungal pests, which comprises treating the fungal pests, their environment, or the seed, plants, areas, materials or spaces to be kept free from them with an effective amount of a compound of the general formula I as claimed in claim I or of a composition as claimed in claim 1.

6. A method as claimed in claim 5, which comprises carrying out the treatment with a compound I from the following table or with a composition as claimed in claim 1 comprising this compound I: 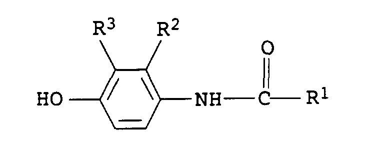
| No. | R³ | R² | R¹ |
|---|---|---|---|
| 1 | Cl | Cl | 2-CH₃-bicyclo-[2.2.1]-heptan-2-yl |
| 2 | Cl | CH₃ | 2-CH₃-bicyclo-[2.2.1]-heptan-2-yl |
| 3 | Cl | Cl | 2-CH₃-bicyclo-[2.2.1]-hepten-2-yl |

## Revendications

1. Produit approprié pour la lutte contre les champignons nuisibles, contenant une quantité efficace d'un dérivé de p-hydroxyaniline de formule générale I 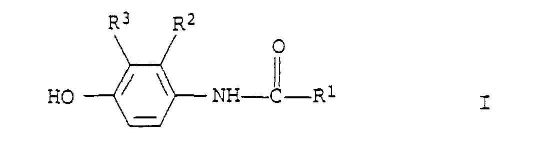 dans laquelle les restes ont la signification suivante:
R¹ bicycloalkyle en C₆-C₁₅ ou bicycloalcényle en C₇-C₁₅, tandis que ces restes peuvent être partiellement ou totalement halogénés et/ou porter un à cinq des groupes suivants: alkyle en C₁-C₄, halogénoalkyle en C₁-C₄, alcoxy en C₁-C₄, halogénoalcoxy en C₁-C₄ et aryle, qui peut être partiellement ou totalement halogéné et/ou porter un à trois des substituants suivants: nitro, cyano, alkyle en C₁-C₄, halogénoalkyle en C₁-C₄, alcoxy en C₁-C₄, halogénoalcoxy en C₁-C₄ et alkylthio en C₁-C₄;
R² et R³, indépendamment l'un de l'autre, halogéno, alkyle en C₁-C₄, halogénoalkyle en C₁-C₄, alcoxy en C₁-C₄ ou halogénoalcoxy en C₁-C₄.

2. Utilisation des composés de formule générale I selon la revendication 1 pour la lutte contre les champignons nuisibles.

3. Utilisation des produits selon la revendication 1 pour la lutte contre les champignons nuisibles.

4. Utilisation des composés de formule générale I selon la revendication 1 pour la préparation de produits appropriés pour la lutte contre les champignons nuisibles.

5. Procédé pour la lutte contre les champignons nuisibles, caractérisé par le fait qu'on traite les champignons nuisibles, leur biotope ou les semences, plantes, surfaces, matériaux ou espaces à tenir à l'abri de ceux-ci, avec une quantité efficace d'un composé de formule générale I selon la revendication 1 ou d'un produit selon la revendication 1.

6. Procédé selon la revendication 5, caractérisé par le fait qu'on effectue le traitement avec un composé I du tableau suivant ou un produit selon la revendication 1 contenant ce composé I: 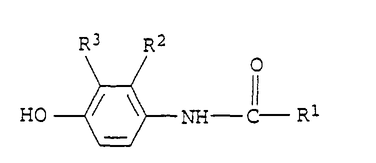
| No | R³ | R² | R¹ |
|---|---|---|---|
| 1 | Cl | Cl | 2-CH₃-Bicyclo- [2.2.1] -heptan - 2 -yle |
| 2 | Cl | CH₃ | 2 - CH₃ -Bicyclo- [2.2.1] -heptan-2-yle |
| 3 | Cl | Cl | 2 -CH₃ -Bicyclo- [2.2.1] -hepten-2-yle |
